# EUROPEAN PATENT SPECIFICATION

(11) **EP 4 085 829 B1**
(45) Date of publication and mention of the grant of the patent: **18.03.2026**
(21) Application number: 21172512.2
(22) Date of filing: 06.05.2021
(51) Int. Cl.: A61B 5/0537, A61B 5/145

(54) **ANALYTE SENSOR AND A METHOD FOR ITS PRODUCING**
ANALYTSENSOR UND VERFAHREN ZU DESSEN HERSTELLUNG
CAPTEUR D'ANALYTE ET SON PROCÉDÉ DE PRODUCTION

(43) Date of publication of application: 09.11.2022
(60) Divisional application: 26155373.9
(73) Proprietor: Roche Diabetes Care GmbH, 68305 Mannheim (DE); F. Hoffmann-La Roche AG, 4070 Basel (CH)
(72) Inventor: SLIOZBERG, Kirill, 68305 Mannheim (DE); STECK, Alexander, 68305 Mannheim (DE)
(74) Representative: Altmann Stößel Dick Patentanwälte PartG mbB

(56) References cited:
- WO-A1-2010/099507
- US-A1- 2003 146 110
- US-A1- 2009 156 920
- US-A1- 2010 025 238
- US-A1- 2011 230 735

## Description

### Field of the invention

The present invention relates to an analyte sensor for determining at least one analyte and a method for producing such an analyte sensor. The analyte sensor may, primarily, be used for determining of a concentration of at least one analyte in a body fluid, in particular of a blood glucose level. However, further applications may also be feasible.

### Related art

Determining of a concentration of one or more analytes in a body fluid, in particular of a blood glucose level, plays an important role in the prevention and treatment of various diseases. Without restricting further possible applications, the invention is described in the following with reference to glucose in an interstitial fluid. However, the invention can also be applied to other types of analytes. Blood glucose monitoring may, specifically, be performed by using electrochemical analyte sensors besides optical measurements. Examples of electrochemical analyte sensors for measuring glucose, specifically in blood or other body fluids, are known from US 5,413,690 A, US 5,762,770 A, US 5,798,031 A, US 6,129,823 A or US 2005/0013731 A1.

WO 2012/045425 A1 discloses an electrochemical sensor that employs multiple electrode areas that are exposed for contact with a body fluid, e.g., when the sensor is inserted subcutaneously into a patient's skin. The exposed electrode areas are arranged symmetrically, such that a symmetrical potential distribution is produced when an AC signal is applied to the sensor. In a particular embodiment, only one reference electrode is covered with Ag/AgCl while the other reference electrodes remain blank.

US 9,936,909 B2 discloses devices for measurement of an analyte concentration, e.g., glucose in a host. Herein, a combined counter/reference electrode formed from silver/silver chloride is used. Further, laser ablation is used to expose the electroactive surfaces. European patent application 20 162 098.6, filed March 10, 2020, discloses a fast transient measurement method for determining membrane properties in an analyte sensor. The fast transient measurement method as disclosed therein can, particularly, be used for measuring an equivalent series resistance (ESR) of the analyte sensor, wherein the measured ESR, may typically, comprise a resistance of a membrane comprised by the analyte sensor. Herein, an ionic conductivity of the membrane may correlate with a permeability of the membrane for an analyte to be determined and, consequently, with a sensitivity of the analyte sensor. The measured ESR can be used to correct a sensitivity shift in the analyte sensor, such as caused by a varying temperature, but may also be used to detect a mechanical load at an insertion site of the sensor in a body of a user, specifically, since the permeability of the membrane may, in particular, depend on a temperature, a chemical surrounding, or a mechanical load of the membrane but not on the dimensions of the membrane.

Apart from the resistance of the membrane, the measured ESR may, further depend on a geometry of the analyte sensor, especially a size of electrodes, or a distance between the electrodes, as well as on at least one coating as applied to the electrodes which can be considered as a fixed portion of the ESR. By subtracting the fixed portion of ESR from the measured ESR a variable portion of the ESR may be obtained which can, mostly, be assigned to membrane effects.

However, an analyte sensor that comprises a protective coating which is applied to isolate a major portion of an electrode in order to minimize a surface area of the electrode which is exposed to the body fluid comprising the analyte may, typically, lead to an increase in the measured ESR. Consequently, the variable portion of the ESR which can be assigned to membrane effects can, in particular, be considerably smaller compared to the fixed portion of the ESR. A reduced range of signal vs. background diminishes an effective resolution when determining membrane properties in the analyte sensor from the measured ESR. As observed, the measured ESR strongly depends on the surface area of the electrode which is exposed to the body fluid comprising the analyte, resulting in considerably different values for the background arising from inhomogeneities during sensor production. As further observed, the measured ESR can, in addition, not be used for a considerable amount of time due to a considerably long running-in period of the measured ESR.

European patent application 20 184 466.9, filed July 7, 2020, discloses an analyte sensor comprising a substrate, at least one working electrode, at least one second electrode and a membrane, wherein the membrane is located on top of the at least one second electrode. European patent application 20 207 218.7, filed November 12, 2020, discloses a method for producing an analyte sensor, an analyte sensor obtainable by this method, and a use of the analyte sensor. The method comprises the steps of: a) providing a first substrate having a first side, and a second side, wherein the second side has a first layer comprising a first conductive material; b) providing a second substrate having a first side, wherein the first side has a second layer comprising a second conductive material, and a second side, wherein second side has a third layer comprising a third conductive material; c) applying a layer of an conductive preparation onto at least one of the first side of the first substrate or the third layer or a portion thereof, wherein the conductive preparation comprises a plurality of conductive particles, and at least one polymeric binder; d) laminating the first side of the first substrate with the second side of the second substrate; and e) obtaining the analyte sensor.

WO 2010/099507 A1 discloses methods and systems for providing continuous analyte monitoring including in vivo sensors that do not require any user calibration during in vivo use are provided. Also provided are methods and devices including continuous analyte monitoring systems that include in vivo sensors which do not require any system executed calibration or which do not require any factory based calibration, and which exhibit stable sensor sensitivity characteristics. Methods of manufacturing the no calibration sensors and post manufacturing packaging and storage techniques are also provided.

### Problem to be solved

It is therefore an objective of the present invention to provide an analyte sensor for determining at least one analyte and a method for producing an analyte sensor for determining at least one analyte, which at least partially avoid the shortcomings of known analyte sensors and related methods and which at least partially address the above-mentioned challenges.

In particular, it is desired to decrease a fixed portion for a measured equivalent series resistance (ESR) of the analyte sensor and to diminish a variation of the measured ESR over a plurality of analyte sensors when applying a fast transient measurement method that can be used for determining membrane properties of the analyte sensor, thereby also reducing an observable running-in period with regard to the measured ESR.

### Summary of the invention

This problem is solved by an analyte sensor for determining at least one analyte and a method for producing an analyte sensor for determining at least one analyte having the features of the independent claims. Preferred embodiments of the invention, which may be implemented in an isolated way or in any arbitrary combination, are disclosed in the dependent claims and throughout the specification.

In a first aspect of the present invention, an analyte sensor for determining at least one analyte is disclosed, wherein the analyte sensor comprises:
- a substrate;
- a working electrode and a conductive layer located on different sites on the substrate;
- a silver comprising layer partially covering the conductive layer; and
- a protective layer covering
   o the silver comprising layer fully apart from at least one area accessible to at least one body fluid comprising the at least one analyte; and
   o a portion of the conductive layer
wherein the portion of the conductive layer (124) covered by the protective layer (128) is of 20 % to 80 %.

As generally used, the term "analyte sensor" refers to an arbitrary device being configured to perform a detection of an analyte by acquiring at least one measurement signal. As particularly preferred, the analyte sensor may be a partially implantable analyte sensor which may, particularly, be adapted for performing the detection of the analyte in a body fluid of a user in a subcutaneous tissue, particularly in an interstitial fluid. As used herein, the term "partially implantable analyte sensor" refers to an analyte sensor designated to be introduced into the body tissue of a patient or user in a fashion that a first portion of the implantable analyte sensor may be received by the body tissue while a further portion may not be received by the body tissue. For this purpose, the analyte sensor may comprise an insertable portion. Herein, the term "insertable portion" generally refers to a part or component of the analyte sensor which is configured to be insertable into an arbitrary body tissue. Other parts or components of the analyte sensor, in particular contact pads, may remain outside of the body tissue.

As generally used, both terms "user" and "patient" refer to a human being or an animal, independent from the fact that the human being or animal, respectively, may be in a healthy condition or may suffer from one or more diseases. As an example, the user or the patient may be a human being or an animal suffering from diabetes. However, additionally or alternatively, the invention may be applied to other types of users, patients or diseases.

As further used herein, the term "body fluid", generally, refers to a fluid, in particular a liquid, which is typically present in a body or a body tissue of the user or the patient and/or may be produced by the body of the user or the patient. Preferably, the body fluid may be selected from the group consisting of blood and interstitial fluid. However, additionally or alternatively, one or more other types of body fluids may be used, such as saliva, tear fluid, urine or other body fluids. During the detection of the at least one analyte, the body fluid may be present within the body or body tissue. Thus, the analyte sensor may, specifically, be configured for detecting the at least one analyte within the body tissue.

As further used herein, the term "analyte" refers to an arbitrary element, component, or compound being present in the body fluid, wherein the presence and/or the concentration of the analyte may be of interest to the user, the patient, to medical staff, such as a medical doctor. In particular, the analyte may be or may comprise at least one arbitrary chemical substance or chemical compound which may participate in the metabolism of the user or the patient, such as at least one metabolite. As an example, the at least one analyte may be selected from the group consisting of glucose, cholesterol, triglycerides, lactate. Additionally or alternatively, however, other types of analytes may be used and/or any combination of analytes may be determined. The detection of the at least one analyte specifically may, in particular, be an analyte-specific detection. Without restricting further possible applications, the present invention is described herein with particular reference to a monitoring of glucose in an interstitial fluid.

In particular, the analyte sensor may be an electrochemical sensor. As used herein, the term "electrochemical sensor" refers to an analyte sensor which is adapted for a detection of an electrochemically detectable property of the analyte, such as an electrochemical detection reaction. Thus, for example, the electrochemical detection reaction may be detected by applying and comparing one or more electrode potentials. Specifically, the electrochemical sensor may be adapted to generate the at least one measurement signal which may, directly or indirectly, indicate a presence and/or an extent of the electrochemical detection reaction, such as at least one current signal and/or at least one voltage signal. The measurement may be a qualitative and/or a quantitative measurement. Still, other embodiments are feasible.

The electrochemical sensor as used herein is arranged in a fashion of an electrochemical cell employing at least one pair of electrodes. As generally used, the term "electrode" refers to an element which is adapted to contact the body fluid, either directly or via at least one semi-permeable membrane or layer, wherein each electrode is embodied in a fashion that an electrochemical reaction occurs at at least one surface of the electrode. In particular, the electrodes may be embodied in a manner that oxidative processes and/or reductive processes may take place at selected surfaces of the electrodes. Generally, the term "oxidative process" refers to a first chemical or biochemical reaction during which an electron is released from a first substance, such as an atom, an ion, or a molecule, that is oxidized thereby. A further chemical or biochemical reaction by which a further substance may accept the released electron is, generally, denominated by the term "reductive process". Together, the first reaction and the further reaction may also be denominated as a "redox reaction". As a result, an electrical current, which relates to moving electrical charges, may be generated hereby. Herein, a detailed course of the redox reaction may be influenced by an application of an electrical potential.

Further, each electrode comprises an electrically conductive material. As generally used, the term "electrically conductive material" refers to a substance which is designed for conducting an electrical current through the substance. For this purpose, a highly conductive material having a low electrical resistance is preferred, in particular to avoid a dissipation of electrical energy carried by the electrical current within the substance. In general, the electrically conductive material may be selected from a noble metal, especially gold; or from an electrically conductive carbon material; however, further kinds of conductive materials may also be feasible.

As further used herein, the term "determining" relates to a process of generating at least one representative result, in particular, by evaluating the at least one measurement signal as acquired by the analyte sensor. Herein, the term "evaluating" may refer to an application of methods for displaying the at least one measurement signal and deriving the at least one representative result therefrom. The at least one measurement signal may, specifically, be or comprise at least one electronic signal, such as at least one voltage signal and/or at least one current signal. The at least one signal may be or may comprise at least one analogue signal and/or may be or may comprise at least one digital signal. Especially in electrical systems, it may be required to apply a prespecified signal to a specific device in order to be able to record the desired measurement signal. By way of example, measuring a current signal may require the application of a voltage signal to the device, or vice-versa.

As further used herein, the term "monitoring" refers to a process of continuously acquiring data and deriving desired information therefrom without user interaction. For this purpose, a plurality of measurement signals are generated and evaluated, wherefrom the desired information is determined. Herein, the plurality of measurement signals may be recorded within fixed or variable time intervals or, alternatively or in addition, at an occurrence of at least one prespecified event. In particular, the analyte sensor as used herein may, especially, be configured for a continuous monitoring of one or more analytes, in particular of glucose, such as for managing, monitoring, and controlling a diabetes state.

In general, the analyte sensor may comprise a sensor body, in particular, a substrate. As used herein, the term "substrate" refers to an arbitrary element designed to carry one or more other elements disposed thereon or therein. Specifically, the substrate may be flexible and/or deformable. In particular, the substrate may be bendable. Particularly preferred, the substrate may be a planar substrate. As generally used, the term "planar" refers to a body comprising extensions in two dimensions, typically denoted as "surface" of the planar body, which exceed the extension in a third dimension, usually denoted as "thickness" of the planar body, by a factor of 2, at least a factor of 5, at least a factor of 10, or even at least a factor of 20 or more. As an example, the substrate may have a thickness of 50 µm to 1 mm, specifically of 80 µm to 500 µm, such as 110 µm to 250 µm. Using a planar substrate may, particularly, facilitate providing a flat sensor. As generally used, the term "flat sensor" refers to a particular type of analyte sensor which comprises a planar substrate that provides a carrier for the further elements, preferably the electrodes, of the analyte sensor to be provided, preferably, in form of layers, directly or indirectly, deposited on the substrate.

The substrate may, specifically, have an elongated shape, such as a strip shape or a bar shape; however, other kinds of shapes may also be feasible. As generally used, the term "elongated shape" indicates that each surface of the planar body has an extension in a direction along the elongation which exceeds an extension perpendicular hereto by at least a factor of 2, at least a factor of 5, at least a factor of 10, or even at least a factor of 20 or more.

Specifically, the planar substrate may have a first side and a second side, wherein the first side and the second side differ from each other. As generally used, the term "side" refers to a surface of the sensor body. Herein, the terms "first" and "second" are considered as description without specifying an order and without excluding an embodiment in which other elements of the same kind may be present. In a preferred embodiment, the first side and the second side may be positioned in an opposite fashion with respect to each other.

The substrate may, preferably, be or comprise at least partially, preferably completely, at least one electrically insulating material, especially in order to avoid unwanted currents between electrically conducting elements as carried by the substrate. In a preferred embodiment, the at least one electrically insulating material may be selected from the group consisting of an insulating epoxy resin, a polycarbonate, a polyester, a polyvinylchloride, a polyurethane, a polyether, a polyethylene, a polyamide, a polyimide, a polyacrylate, a polymethacrylate, a polytetrafluoroethylene or a copolymer thereof, and alumina; however, other kinds of electrically insulating materials may also be feasible. Herein, a suitable polyester may be polyethylene terephthalate (PET).

According to the present invention, the analyte sensor comprises a working electrode and at least one further electrode, wherein the working electrode and the at least one further electrode are located on different sites on the substrate, preferably on the first side and on the second side of the substrate, wherein the first side and the second side may, preferably, be positioned on opposite sides of the substrate. In a particularly preferred embodiment, the working electrode and the at least one further electrode may be located on opposing sides of the substrate in a manner that a geometrical projection of the site of the working electrode onto the side of the substrate on which the at least one further electrode may be located does not result in overlap between the geometrical projection of the site of the working electrode and the site of the at least one further electrode. However, placing the working electrode and the at least one further electrode on different sites on the same side of the substrate, such as both on the first side or the second side of the substrate, may also be feasible.

The at least one further electrode may, preferably, be a counter electrode, a reference electrode and/or a combined counter/reference electrode. As particularly preferred, the at least one further electrode may be or comprise only a single combined counter/reference electrode, specifically in order to provide an analyte sensor having a considerably small spatial expansion particularly configured to be used as implantable sensor. If the analyte sensor has only two electrodes, it typically comprises a single working electrode and the single combined counter/reference electrode. Herein, the working electrode may have a reagent layer comprising a biorecognition component, specifically an enzyme having a redox active enzyme co-factor adapted to support an oxidation of the analyte in the body fluid. In a particularly preferred embodiment, each electrode may have a lateral extension of 0.2 mm to 10 mm, preferably of 0.5 mm to 5 mm, more preferred of 0.7 mm to 2 mm, and a thickness of 1 µm to 10 µm, preferably of 2 µm to 5 µm. However, further embodiments of the electrodes may also be feasible.

As indicated above, the at least one further electrode comprises a conductive layer, a silver comprising layer which partially covers the conductive layer; and a protective layer which covers the silver comprising layer and a portion of the conductive layer. Further portions of a particular side of the substrate may be covered, partially or completely, by an insulating layer. As used herein, the term "layer" refers to a volume that comprises a material having extensions in two lateral dimensions, typically denoted as "lateral extension" or simply "extension" of the layer, which exceed the extension in a third dimension, usually denoted as "thickness" of the layer, by a factor of 2, at least a factor of 5, at least a factor of 10, or even at least a factor of 20 or more. Herein, the layer may be carried by the respective substrate, in particular, in order to provide stability and integrity to the layer. The layer may, specifically, have an elongated shape, such as a strip shape or a bar shape; however, other kinds of shapes may also be feasible. As described below in more detail, each layer may, preferably, be produced in an additive process by applying a desired material to the substrate or to an already deposited layer; however, using a further process may also be feasible. For further details, reference can be made to the description of the method below. The conductive layer is an electrically conductive layer. As generally used, the term "electrically conductive" refers to a property of a substance of conducting an electrical current through the substance. Preferably, the conductive layer may comprise an electrically conductive material. As already defined above, the term "electrically conductive material" refers to a substance which is designed for conducting an electrical current through the substance. The electrically conductive material may, as particularly preferred, be selected from an electrically conductive carbon material; however, a further kind of conductive materials, such as a noble metal, especially gold, may also be feasible.

In a further embodiment, the conductive layer may be or comprise a structured layer, such as disclosed in European patent application 20207218.7, filed November 12, 2020. Accordingly, the electrically conductive material may be selected from the group consisting of gold, nickel, platinum, palladium, carbon, carbon paste, polyaniline and poly-3,4-ethylenedioxythiophene (PEDOT), particularly preferred, the at least one electrically conductive material is selected from the group consisting of gold, carbon, and carbon paste. More preferably, the at least one electrically conductive material consists essentially of gold and/or carbon and/or carbon past.

Further, the at least one further electrode has a silver comprising layer. As used herein, the term "silver comprising layer" refers to a particular type of layer which comprises a silver comprising substance, specifically selected from at least one of elemental silver (Ag) and a silver comprising compound, wherein the silver comprising compound may, preferably, be silver chloride (AgCl). In a preferred embodiment, the silver comprising layer may only comprise AgCl at a time when the analyte sensor is produced, wherein no elemental Ag may be added at that time. However, during use of the analyte sensor, elemental Ag may then be formed from the AgCl in a fashion that, during use, the analyte sensor may, subsequently, comprise Ag/AgCl. An analyte sensor which has a silver comprising layer that comprises Ag/AgCl is, particularly, preferred.

In a particular embodiment, the Ag/AgCl may be comprised as a plurality of conductive particles in a binder within the silver comprising layer. As generally used, the term "binder" refers to a substance designated to maintain the plurality of the conductive particles within a preparation at least partially, preferably completely, together. The conductive particles may, in particular, be dispersed within the at least one binder. Especially, the conductive particles may be homogeneously dispersed within the at least one binder. Herein, the at least one binder may be selected from the group consisting of metallic binders, ceramic binders and polymeric binders. Preferred are polymeric binders, in particular physically binding polymer binders and/or chemically binding polymer binders. By way of example, the Ag/AgCl within the silver comprising layer may comprise 50 to 70 wt.-% of Ag, 20 to 40 wt.-% of AgCl and 1 to 20 wt.-% of a binder, wherein each wt.-% is based on a sum of the wt.-% of Ag, AgCl and the binder. However, a further kind of preparation of the silver comprising layer may also be feasible.

As further indicated above, the silver comprising layer partially covers the conductive layer. As used herein, the term "partially" refers to a section of an accessible surface of the conductive layer which is covered by the silver comprising layer, wherein a remaining section of the accessible surface of the conductive layer is not covered by the silver comprising layer. As further used herein, the term "accessible surface" refers to a particular surface of a layer, such as of the silver comprising layer or of the conductive layer, not already adjoining a further surface and, thus, being ready for being covered by a further layer. Herein, the section of the conductive layer which is covered by the silver comprising layer may, preferably, be of 2 % to 50 %, more preferred of 5 % to 40 %, in particular of 10 % to 30 %, of the accessible surface of the conductive layer.

Further, the at least one further electrode comprises a protective layer. As generally used, the term "protective layer" refers to a particular type of layer which is applied in order to control access to a further layer which is partially or fully covered by the protective layer. As used herein, the term "fully" refers to a complete disruption of the access to the further layer, while the term "partially" refers to a selective barrier which provides selective access to the further layer.

In a preferred embodiment, the protective layer can be or comprise a membrane which may comprise a plurality of holes, wherein the holes are designed to provide access to the further layer for the at least one body fluid comprising the at least one analyte. As generally used, the term "holes" refers to an opening and/or perforation in the membrane, thereby allowing a passage of one or more molecules and/or compounds through the holes, whereby the desired selective access to the further layer is provided for the body fluid. Herein, the holes may be distributed in an equal fashion or in an unequal fashion over the aerial expansion of the membrane. Specifically, the holes may exhibit a total area of 0.005 mm² to 0.15 mm², preferably of 0.01 mm² to 0.02 mm². As used herein, the term "total area of holes" relates to the sum of the surface area of the holes as comprised by the membrane. Further, the membrane may have a thickness of 1 µm to 100 µm, preferably of 2 µm to 25 µm, more preferred of 5 µm to 20 µm.

In a particularly preferred embodiment, the protective layer may be a hydrophobic layer. As used herein, the term "hydrophobic" refers to a property of the protective layer in that a protective material as comprised by the protective layer may have a water uptake of 0 to 5 % by weight, preferably of less than 1 % by weight, based on a total weight of the protective material. Herein, the protective material may, preferably, comprise at least one hydrophobic polymer, preferably a thermoplastic hydrophobic polymer. Specifically, the hydrophobic polymer may be selected from the group consisting of thermoplastic polyurethanes (TPU), thermoplastic polyurea, polyethylene, polypropylene, polystyrene, butyl methacrylate polymers (BUMA), polyethylene terephthalate (PET), and UV hardening resins, especially acrylated silicones, acrylated urethanes, acrylated polyesters and acrylated epoxides. In particular, the hydrophobic polymer may be or comprise a thermoplastic polyurethane. However, other types of hydrophobic polymers may also be feasible.

For further details concerning the membrane, specifically with regard to its properties and composition, reference may be made to European patent application 20 184 466.9, filed July 7, 2020, which discloses an analyte sensor comprising a membrane.

In particular accordance with the present invention, the protective layer covers
- the silver comprising layer fully apart from at least one area accessible to at least one body fluid comprising the at least one analyte; and
- a portion of the conductive layer.

Herein, the silver comprising layer can be fully covered apart from at least one area accessible to at least one body fluid comprising the at least one analyte by using the protective layer as disclosed above which comprises the membrane having the plurality of the holes which are designated for providing the desired selective access to the silver comprising layer for the at least one body fluid comprising the at least one analyte. Alternatively or in addition, the silver comprising layer may comprise an exposed cutting edge which may also be designated for providing the desired access to the silver comprising layer for the at least one body fluid comprising the at least one analyte. As used herein, the term "cutting edge" refers to a surface on the silver comprising layer which is accessible for the at least one body fluid comprising the at least one analyte as generated by removing a portion of the protective layer from the surface of the silver comprising layer. In general, the removing of the portion of the protective layer from the surface of the silver comprising layer has been performed when cutting a raw substrate as defined below in more detail into appropriate pieces configured to form the individual analyte sensors. Herein, a cutting process may be applied which may, concurrently, remove the portion of the protective layer from the surface of the silver comprising layer when a cutter may be applied for this purpose. Independently from the selected arrangement, the desired selective access to the silver comprising layer for the at least one body fluid comprising the at least one analyte allows determining the at least one analyte according to the present invention from an interaction of the body fluid with the silver comprising layer.

As indicated above, the protective layer only covers a portion of the conductive layer. As generally used, the term "portion" refers to a section of an accessible surface of the conductive layer which is covered by the protective layer, wherein a remaining section of the accessible surface of the conductive layer is not covered by the protective layer. For the term "accessible surface" reference may be made to the definition as provided above. The portion of the conductive layer as covered by the protective layer is of 20 % to 80 %, preferably of 25 % to 75 %, specifically of 30 % to 70 %, in particular of 40 % to 60 %.

Surprisingly, it could be experimentally demonstrated that leaving the remaining section of the accessible surface of the conductive layer uncovered by the protective layer is capable of drastically reducing the measured equivalent series resistance (ESR) of the analyte sensor when applying a fast transient measurement method, such as described in European patent application 20 162 098.6, filed March 10, 2020, in more detail. As generally used, the term "fast-transient voltage" as refers to at least one alteration of an electrical voltage between two electrodes, wherein the alteration may exhibit fast transient signal flanks, in particular two very steep edges. The electrical voltage as applied during the fast transient measurement method may comprise a signal selected from at least one of a square wave form, a sine wave form, or a non-continuous signal, such as a voltage pulse.

Herein, the term "pulse" refers to a signal having a transient alteration of an amplitude of the signal from a first baseline value, via a rising flank or a falling flank, to a second value, followed by a return, via a falling flank or a rising flank, to the baseline value or at least approximately to the baseline value, wherein the second value may be higher or lower than the baseline value. A pulse duration may be ≤ 50 µs, preferably ≤ 20 µs, more preferred ≤ 10 µs. In particular, the duration of a single pulse may be sufficiently long to record its propagation and may be short enough not to excite the analyte sensor in an electrochemical manner. As further generally used, the term "fast-transient" refers to time range between first and second values of the signal flank, which can, preferably, be 50 ns or below, more preferred 20 ns or below, and may be only limited by electronics such as by an analog-to-digital-converter. A faster flank and a sharper transition to a plateau as constituted by the baseline value or by the second value can increase a resolution between an ohmic part of the resistance and a capacitive part of the capacitance of the analyte sensor.

As indicated above, the fast transient measurement method can, particularly, be used for measuring an equivalent series resistance (ESR) of the analyte sensor, wherein the measured ESR, may typically, comprise a resistance of a membrane comprised by the analyte sensor. Herein, an ionic conductivity of the membrane may correlate with a permeability of the membrane for an analyte to be determined and, consequently, with a sensitivity of the analyte sensor. The measured ESR can be used to correct a sensitivity shift in the analyte sensor, such as caused by a varying temperature, but may also be used to detect a mechanical load at an insertion site of the sensor in a body of a user, specifically, since the permeability of the membrane may, in particular, depend on a temperature, a chemical surrounding, or a mechanical load of the membrane but not on the dimensions of the membrane. As a result, an effective resolution of fast transient measurements can considerably be increased when determining membrane properties in the analyte sensor according to the present invention from the measured ESR.

In a further embodiment, the analyte sensor may comprise an electrically insulating layer, which may cover remaining surfaces of the analyte sensor, specifically a portion of the conductive layer which is not covered by the silver comprising layer and/or the protective layer and which is not part of the accessible surface to the conductive layer that is left free from the protective layer. In particular, the electrically insulating layer may be an electrically insulating varnish, such as a photoresist or a solder resist; however, a further kind of electrically insulating layer may also be feasible. This embodiment may further support protecting the analyte sensor.

In a further aspect of the present invention, a method for producing an analyte sensor for determining at least one analyte, in particular for producing the analyte sensor for determining at least one analyte as described herein, is disclosed. The method comprises the following steps of:
a) providing a raw substrate;
b) applying a conductive layer on the raw substrate;
c) applying a silver comprising layer in a manner that it partially covers the conductive layer;
d) applying a protective layer in a manner that it covers
   o the silver comprising layer; and
   o a portion of the conductive layer such that the portion of the conductive layer (124) covered by the protective layer (128) is of 20 % to 80 %;
e) preparing a working electrode; and
f) cutting the raw substrate to obtain the analyte sensor.

Herein, the indicated steps may, preferably, be performed in the given order, thereby commencing with step a), continuing with steps b), c), d) and e), and finishing with step f). However, as described below in more detail, step e) may also be performed after step f). Further, additional steps, whether described herein or not, may be performed, too.

According to step a), a raw substrate is provided. As used herein, the term "raw substrate" refers to at least one material designated to form a carrier layer to support the working electrode and the at least one further electrode as defined above. From the raw substrate, the substrate of the analyte sensor as described elsewhere herein can be produced, specifically by cutting the raw substrate into appropriate pieces. In particular, the raw substrate may comprise an electrically insulating material. For the electrically insulating material as well as for further properties of the raw substrate, reference may be made to the description of the substrate above. However, a length and a width of the raw substrate may, in general, considerably differ from the length and the width of the substrate as comprised by the completed analyte sensor. Preferably, the raw substrate may have a length of 1 cm to 1 km, more preferred of 10 cm to 100 m, and a width of 1 cm to 10 cm, more preferred of 2 cm to 8 cm. In a preferred embodiment, the raw substrate can be provided as a roll, in particular designated for being used in a roll-to-roll process.

According to step b), a conductive layer, in particular the conductive layer as described elsewhere herein is applied on the raw substrate. As generally used, the term "applying" refers to an additive process by which a desired material is deposited on the substrate or to an already deposited layer; however, using a further process for producing the desired layer may also be feasible. In particular, the additive process may be selected from a known deposition process, including but not limited to silk-screen printing, slot-die coating, sputtering, vacuum evaporation, atomic layer deposition, chemical vapor deposition, spray pyrolysis, electrodeposition, anodization, electro-conversion, electro-less dip growth, successive ionic adsorption and reaction, chemical bath deposition, and a solution-gas interface technique, wherein silk-screen printing or slot-die coating may, particularly, be preferred.

According to step c), a silver comprising layer is applied in a manner that it partially covers the conductive layer. For further details concerning the silver comprising layer and the applying procedure, reference may be made to the description above.

According to step d), a protective layer is applied in a manner that it covers the silver comprising layer; and a portion of the conductive layer. For further details concerning the protection layer and the applying procedure, reference may be made to the description above. As already indicated above, the protective layer may be or comprise a membrane having a plurality of holes designated for providing selective access to the silver comprising layer for the at least one body fluid comprising the at least one analyte.

According to step e), a working electrode is prepared. For further details concerning the working electrode and the applying procedure, reference may be made to the description elsewhere herein.

According to step f), the raw substrate is cut to obtain the analyte sensor. For this purpose, the raw substrate may be cut into sheets, preferably along its width, whereby sensor strips may be formed. Herein, each sensor strip may, preferably, correspond to a single analyte sensor. In addition, the raw substrate can be cut at least once along its length, specifically before or after the raw substrate may be cut along its width. Preferably, the raw substrate may be cut using at least one laser. Consequently, step f) may comprise a laser-cutting process. As already described above in more detail, the cutting may be performed during step f) in a fashion that the silver comprising layer may have an exposed cutting edge designated for providing access to the silver comprising layer for the at least one body fluid comprising the at least one analyte.

In a preferred embodiment, the working electrode may, preferably, be prepared prior to cutting the raw substrate into sheets, specifically by applying an appropriate coating on a side of the raw substrate. Alternatively or in addition, the working electrode may be prepared after cutting the raw substrate into sheets, specifically by generating each individualized analyte sensor by applying an appropriate coating.

At least one further method step, whether disclosed herein or not, may also be performed. In particular, an electrically insulating layer may be applied to cover remaining surfaces of the analyte sensor, specifically a portion of the conductive layer which is not covered by the silver comprising layer and/or the protective layer and which is not part of the accessible surface to the conductive layer that is left free from the protective layer, as described above on more detail.

For further details with regard to the method, reference can be made to the description of the analyte sensor as provided elsewhere herein.

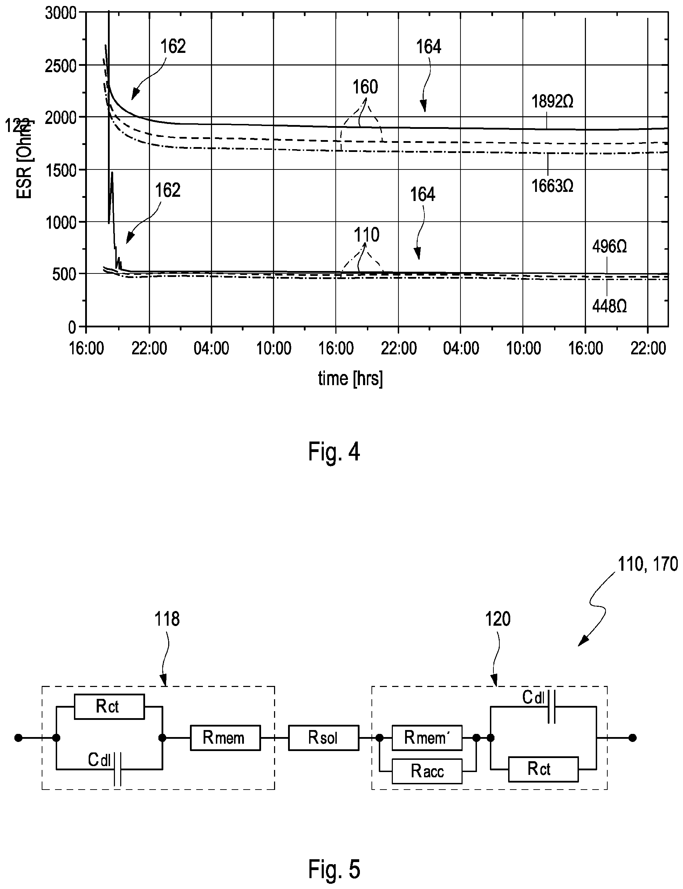

The analyte sensor for determining at least one analyte and the method for producing such an analyte sensor according to the present invention exhibit particular advantages with respect to the prior art. As demonstrated below, particularly in Figure 4, the analyte sensor which comprises a modified electrode arrangement as described herein significantly reduces noise during measurements, thereby increasing a signal-to-noise ratio in fast-transient measurements of the analyte sensor. As further presented in Figure 4, the method for producing the analyte sensor allows considerably higher tolerances during producing of the analyte sensor, mainly owing due to fact that different analyte sensors, such as preferably obtained from a common raw substrate, exhibit a considerably reduced variation of the ESR over the analyte sensors. Furthermore, the modified electrode arrangement as used for the analyte sensor according to the present invention can be produced in an easier manner compared to prior art analyte sensors. In addition, further advantages are conceivable.

As used herein, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. Herein, in most cases, when referring to the respective feature or element, the expressions "at least one" or "one or more" will not be repeated, non-withstanding the fact that the respective feature or element may be present once or more than once.

Further, as used herein, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the person skilled in the art will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

### Short description of the figures

Further details of the invention can be derived from the following disclosure of preferred embodiments. The features of the embodiments can be implemented in an isolated way or in any combination. The invention is not restricted to the embodiments. The embodiments are schematically depicted in the Figures. The Figures are not to scale. Identical reference numbers in the Figures refer to identical elements or functionally identical elements or elements corresponding to each other with regard to their functions.

In the Figures:
- Figure 1: schematically illustrates an aerial view of an analyte sensor according to the present invention;
- Figure 2: schematically illustrates a method for producing an analyte sensor according to the present invention;
- Figure 3: schematically illustrates selected steps for producing an exemplary embodiment of the analyte sensor according to the present invention;
- Figure 4: schematically illustrates recorded values for a temporal development of an equivalent series resistance (ESR) of the analyte sensor according to the present invention compared to a prior art analyte sensor; and
- Figure 5: schematically illustrates an equivalent circuit diagram of the analyte sensor according to the present invention.

### Detailed description of the embodiments

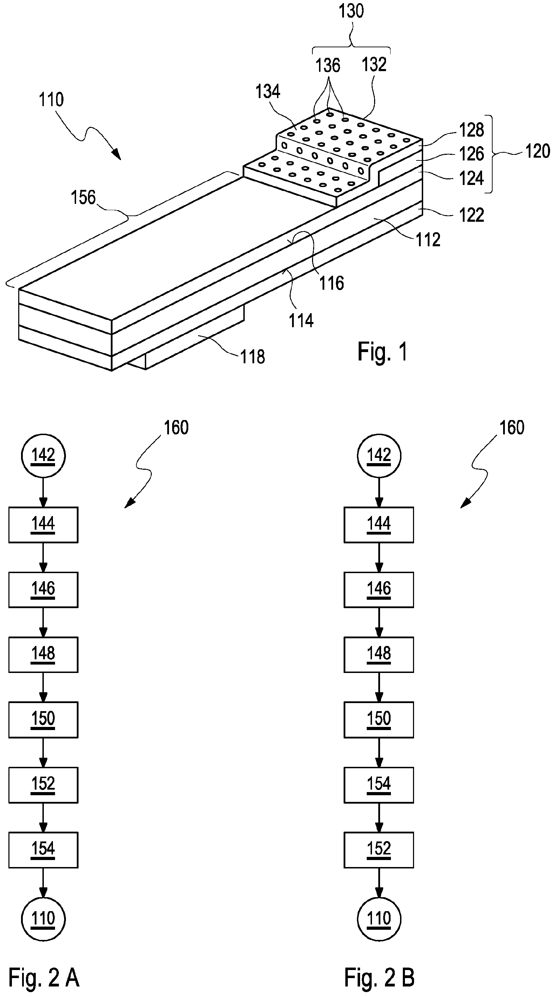

Figure 1 schematically illustrates an aerial view of the analyte sensor 110 according to the present invention. It is particularly emphasized here that the dimensions as used in Figure 1 are not to scale. The analyte sensor 110 may, as depicted there, be a flat sensor that can be partially implantable for continuously monitoring an analyte, in particular by performing a continuous measurement of one or more analytes in a subcutaneous tissue, preferably in a body fluid, especially in an interstitial fluid or in blood. For this purpose, the analyte sensor 110 may be configured to convert the one or more analytes into an electrically charged entity by using an enzyme. Specifically, the one or more analytes may comprise glucose, which may be converted into an electrically charged entity by using at least one of glucose oxidase (GOD) or glucose dehydrogenase (GHD) as the enzyme. However, the analyte sensor 110 may also be applicable to other analytes and/or to other processes for monitoring an analyte.

As illustrated in Figure 1, the analyte sensor 110 comprises an electrically insulating substrate 112. As described above in more detail, the substrate 112 may, in particular, be an elongated planar substrate 112, specifically having a strip shape or a bar shape, which may, preferably, be flexible and/or deformable and/or bendable, and is designated for carrying the further layers as described below. Using the planar substrate 112 may, in particular, facilitate providing the flat sensor. The substrate 112 may comprise at least one electrically insulating material, preferably selected from the group as indicated above, especially in order to avoid unwanted currents between electrically conducting elements carried by the substrate 112.

As further depicted in Figure 1, the planar substrate 112 has a first side 114 and a second side 116, wherein the first side 114 and the second side 116 are positioned in an opposite fashion with respect to each other. In the exemplary embodiment of the analyte sensor 110 as shown in Figure 1, a working electrode 118 is located on the first side 114 of the planar substrate 112 while a further electrode 120 is located on the second side 116 of the planar substrate 112. As further illustrated there, the working electrode 118 and the further electrode 120 are located on the substrate 112 in a manner that a geometrical projection of the site of the working electrode 118 onto the second side 116 of the substrate 112 on which the further electrode 120 is located does not result in overlap between the geometrical projection of the site of the working electrode 118 and the site of the further electrode 120. As an alternative, placing both the working electrode 118 and the further electrode 120 on different sites on the same side of the substrate 112 may also be feasible.

The further electrode 120 may, preferably, be a counter electrode, a reference electrode and/or a combined counter/reference electrode. As particularly preferred, the further electrode may be or comprise a single combined counter/reference electrode, such that the analyte sensor 110 could be considerably small to be used as implantable sensor.

As further depicted in Figure 1, the further electrode 120 comprises a conductive layer 122, 124 which directly covers, preferably fully, both the first side 114 and the second side 116 of the substrate 114. Preferably, the conductive layer 122, 124 may comprise an electrically conductive material, specifically selected from a noble metal, especially gold; or, as particularly preferred, from an electrically conductive carbon material; however, further kinds of conductive materials may also be feasible. As an alternative, the conductive layer 122, 124 may comprise a layered structure, such as described above in more detail.

As further illustrated in Figure 1, a silver comprising layer 126 partially covers the conductive layer 124 on the second side 116 of the substrate 114. As already indicated above, the silver comprising layer 126 may, preferably, comprise Ag/AgCl, which can, in particular, be generated from an original AgCl layer during use of the analyte sensor 110, wherein elemental Ag may be formed from the AgCl over the use of the analyte sensor 110. As further already indicated above, the portion of the conductive layer 124 which may be covered by the silver comprising layer 124 can, preferably, be of 5 % to 30 %, more preferred of 10 % to 25 %, in particular of 15% to 20%, of the surface of the conductive layer 124 on the second side 116 of the substrate 114 that is located in an opposite fashion with respect to the second side 116 of the substrate to which the conductive layer 124 is applied to.

As further depicted in Figure 1, a protective layer 128 covers the silver comprising layer 126 and a portion of the conductive layer 124 on the second side 116 of the substrate 114. As further already indicated above, the portion of the conductive layer 124 on the second side 116 of the substrate 114 which is covered by the protective layer 128 is of 20 % to 80 %, preferably of 25 % to 75 %, specifically of 30 % to 70 %, in particular of 40 % to 60 %. As illustrated in Figure 4, it could be experimentally demonstrated that leaving the remaining section of the accessible surface of the conductive layer 124 on the second side 116 of the substrate 114 uncovered by the protective layer 128 can, in a surprising manner, drastically reduce the measured equivalent series resistance (ESR) of the analyte sensor 110.

As further illustrated in Figure 1, the silver comprising layer 126 is fully covered by the protective layer 128 apart from an accessible area 130 which provides access to the silver comprising layer 126 for the body fluid that comprises the one or more analytes. As schematically depicted here, the silver comprising layer 126 may comprise an exposed cutting edge 132 which provides the desired access to the silver comprising layer 126 for the body fluid comprising the one or more analytes. Herein, the cutting edge 132 may be a surface on the silver comprising layer 126 generated by removing a portion of the protective layer 128 from the surface of the silver comprising layer 126 when performing a cutting process during which a raw substrate is cut into appropriate pieces configured to form the individual analyte sensors 110. As further schematically depicted here, the protective layer 128 may, alternatively or in addition, be a membrane 134 which comprises a plurality of holes 136, wherein the holes 136 are designed to provide access to the silver comprising layer 126 for the body fluid comprising the one or more analytes. Independently from the selected arrangement, the desired selective access to the silver comprising layer 126 for the body fluid comprising the one or more analytes allows determining the one or more analytes from an interaction of the body fluid with the silver comprising layer 126.

In a further embodiment (not depicted here), an electrically insulating layer may cover a further portion of the conductive layer 124, specifically a portion of the conductive layer 124 which is not covered by the silver comprising layer 126 and/or the protective layer 128 and which is not part of the accessible surface 130 to the conductive layer 124 that is left free from the protective layer 128. Herein, the electrically insulating layer may be or comprise an electrically insulating varnish; however, other electrically insulating materials may also be feasible.

Figure 2 schematically illustrates a method 140 for producing the analyte sensor 110 according to the present invention.

A raw substrate 142 is provided in a providing step 144 according to step a). As already indicated above, the raw substrate 142 has the same insulating material and the same thickness as the substrate 112 but differs from the substrate 112 by a length and a width. The individual analyte sensors 110 each comprising the substrate 112 may be isolated from the raw substrate 142 by using a cutting process as described above in more detail. For ease of processing, the raw substrate 142 may, preferably, be designated for being used in a roll-to-roll process and may, in particular, be provided as a roll.

The conductive layer 122, 124 is applied in a first applying step 146 according to step b) to the raw substrate 142, preferably, in a fashion that it may directly cover, preferably fully, both the first side 114 and the second side 116 of the substrate 114.

The silver comprising layer 126 is applied in a second applying step 148 according to step c) in a manner that it partially covers the conductive layer 124 as applied to the second side 116 of the substrate 114 in a fashion as described above in more detail.

The protective layer 128 is applied in a third applying step 150 according to step d) in a manner that it covers a portion of the conductive layer 124 and the silver comprising layer 126 fully apart from the accessible area 130 which provides access to the silver comprising layer 126 for the body fluid that comprises the one or more analytes as further described above in more detail.

The working electrode 118 is prepared in a preparing step 152 according to step e), preferably on an opposite side of the raw substrate 142.

The raw substrate 142 is cut in a cutting step 154 according to step f) into appropriate pieces, specifically by using a laser cutting process, in order to obtain the desired analyte sensor 110.

As schematically illustrated in Figure 2A, the working electrode 118 may be prepared in the preparing step 152 prior to the cutting of the raw substrate 142 in the cutting step 154. However, in an alternative embodiment as depicted in Figure 2B, the working electrode 118 may be prepared in the preparing step 152 after the cutting of the raw substrate 142 in the cutting step 154. For respective procedures and involved advantages of the corresponding arrangements of the preparing step 152 and the cutting step 154, reference may be made to the corresponding description above.

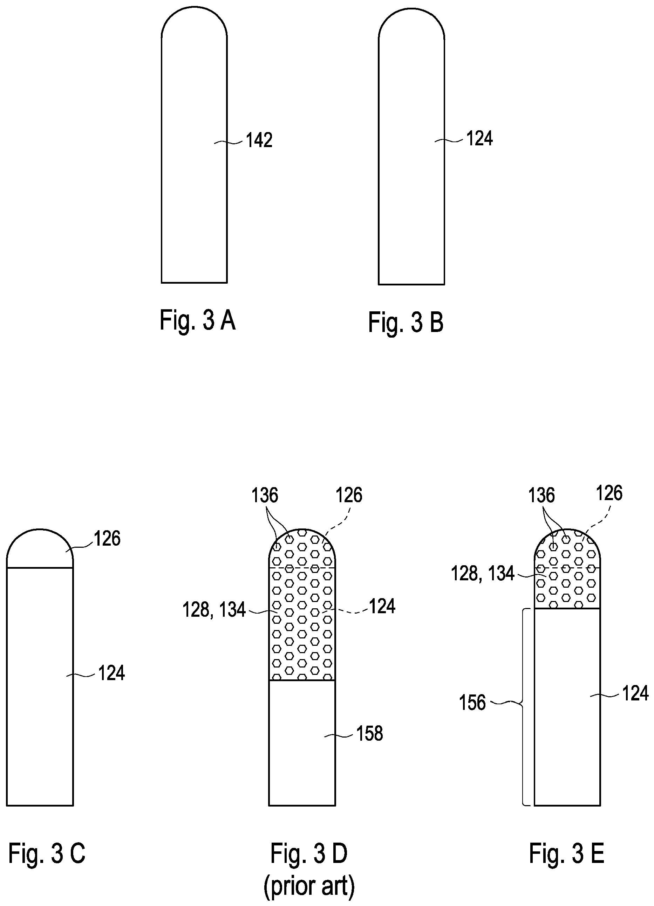

Figure 3 schematically illustrates selected method steps for producing an exemplary embodiment of the method 140 for producing the analyte sensor 110. In a top view, the selected method steps are shown for a single analyte sensor; however, in practice the method may, typically, comprise producing a plurality of analyte sensors 110 in a simultaneous fashion on the single raw substrate 142.

As depicted in Figure 3A, the raw substrate 142, which comprises a body of polyethylene terephthalate (PET) that may, preferably, be plain or, as an alternative (not depicted here), coated with a Au layer, in particular having a thickness of 50 nm to 200 nm, specifically of 100 nm, is provided according to the providing step 144. In particular, the raw substrate 142 as illustrated here is identical with the substrate 112 of the analyte sensor 110 since, as indicated above, only a single analyte sensor 110 is produced in this exemplary embodiment.

As depicted in Figure 3B, the raw substrate 142 as provided according to the providing step 144 is covered, preferably fully, with carbon paste which may form the conductive layer 124 according to the first applying step 146.

As depicted in Figure 3C, a portion of the conductive layer 124 as provided during the first applying step 146 is covered with an Ag/AgCl paste in order to form the silver comprising layer 126 according to the second applying step 148.

Figure 3D depicts a process step as known from prior art in which a thermoplastic polyurethane (TPU) is applied as the protective layer 128 in a manner that it fully covers both the conductive layer 124 and the silver comprising layer 126 fully apart from the accessible area 130 which provides access to the silver comprising layer 126 for the body fluid that comprises the one or more analytes. Remaining sections on the same side of the raw substrate 142 are coated with an insulating layer 158. As a result, a prior art analyte sensor 160 is obtained, in which the accessible area 130 amounted to about 0.025 mm².

In contrast hereto, Figure 3E depicts the third applying step 150 in which the thermoplastic polyurethane (TPU) is applied as the protective layer 128 in a manner that it covers the silver comprising layer 126 fully apart from the accessible area 130 which provides access to the silver comprising layer 126 for the body fluid that comprises the one or more analytes but leaves an accessible surface 156 to the conductive layer 124 which is maintained free from the protective layer 128. As a result, the exemplary analyte sensor 110 according to the present invention is obtained, in which the accessible area 130, again, amounted to about 0.025 mm². However, the accessible surface 156 to the conductive layer 124 which was left free from the protective layer 128 amounted to about 1.4 mm². Consequently, a size of the accessible surface 156 to the conductive layer 124 clearly exceeds the size of the accessible area 130 to the silver comprising layer 126, in particular by a factor of at least 10, preferably of at least 25, more preferred of at least 50.

Figure 4 illustrates measured values of an equivalent series resistance (ESR) in Ohms over a period of time covering 2,25 days of
- three individual exemplary analyte sensors 110 according to the present invention having a setup as schematically depicted in Figure 3E; and
- three individual exemplary prior art analyte sensors 160 having a setup as schematically depicted in Figure 3D.

As demonstrated in Figure 4, compared to the three exemplary prior art analyte sensors 160 the three exemplary analyte sensors 110 according to the present invention exhibit after a running-in period 162 during a measuring period 164
- a considerably decreased value for the ESR;
- a considerably reduced variation of the ESR over the corresponding exemplary analyte sensors 110; and
- a considerably diminished duration of the running-in period 162.

These advantageous observations can, in particular, be explained by a reduced impact of the exposed cutting edges 132, which vary from one analyte sensor 110 to another analyte sensor 110, mainly due to process tolerances, as well as of the holes 136 comprised by the membrane 134 covering the silver comprising layer 126 on the value of the ESR. In a similar fashion, the impact of the initial water uptake which induces the generally observed increase of the values of the ESR during the running-in period 162 is reduced by the predominant contribution of the accessible surface 156 to the ESR.

These effects are summarized in a model as schematically illustrated in Figure 5 which comprises an equivalent circuit diagram 170 that represents the analyte sensor 110 according to the present invention. During a fast-transient measurement of the analyte sensor 110, a capacity of a double layer C_{dl} acts as a shunt capacitor in both the working electrode 118 and the further electrode 120 and can, thus, be disregarded when measuring the equivalent series resistance (ESR) of the analyte sensor 110. As a result, the ESR of the analyte sensor 110 in this model may only depend on a sum of
- a membrane resistance Rₘₑₘ in the working electrode 118;
- a solution resistance Rₛₒₗ; and
- a parallel resistance in the further electrode 120 as generated by a membrane resistance R_{mem'} and an additional resistance R_{acc} which can be attributed to the accessible surface 156 to the conductive layer 124 as illustrated in Figures 1 and 3F.

Owing to the well-known relationship that a value for a total resistor of a plurality of parallel resistors is always lower than the value of each of the resistors, the total ESR, consequently, exhibits a lower value in the analyte sensor 110 according to the present invention as represented by the equivalent circuit diagram 170 of Figure 5. As a result, the model can at least explain the considerably decreased value for the ESR observed in the measurements as illustrated in Figure 4.

### List of reference numbers

- 110: analyte sensor
- 112: substrate
- 114: first side
- 116: second side
- 118: working electrode
- 120: further electrode
- 122: conductive layer
- 124: conductive layer
- 126: silver comprising layer
- 128: protective layer
- 130: accessible area
- 132: cutting edge
- 134: membrane
- 136: holes
- 140: method for producing an analyte sensor
- 142: raw substrate
- 144: providing step
- 146: first applying step
- 148: second applying step
- 150: third applying step
- 152: preparing step
- 154: cutting step
- 156: accessible surface
- 158: insulating layer
- 160: prior art analyte sensor
- 162: running-in period
- 164: measuring period
- 170: equivalent circuit diagram

## Claims

1. An analyte sensor (110) for determining at least one analyte, comprising,
- a substrate (112);
- a working electrode (118) and a conductive layer (122, 124) located on different sites on the substrate (112);
- a silver comprising layer (126) partially covering the conductive layer (124); and
- a protective layer (128) covering
o the silver comprising layer (126) fully apart from at least one area (130) accessible to at least one body fluid comprising the at least one analyte; and
o a portion of the conductive layer (124);
**characterized in**
**that** the portion of the conductive layer (124) covered by the protective layer (128) is of 20 % to 80 %.

2. The analyte sensor (110) according to claim 1, wherein the conductive layer (124), the silver comprising layer (126) and the protective layer (128) form at least one further electrode (120) selected from a group consisting of a counter electrode, a reference electrode and a combined counter/reference electrode,

3. The analyte sensor (110) according to claim 2, wherein the at least one further electrode (120) is or comprises a single combined counter/reference electrode.

4. The analyte sensor (110) according to any one of claims 2 or 3, wherein the working electrode (118) is located on a first side (114) of the substrate (112) and wherein the at least one further electrode (120) is located on a second side (116) of the substrate (112), wherein the first side (114) and the second side (116) of the substrate (112) are positioned opposite each other.

5. The analyte sensor (110) according to claim 4, wherein the working electrode (118) and the at least one further electrode (120) are located on the substrate (112) in a manner that a geometrical projection of a site of the working electrode (118) onto the second side (116) of the substrate (112) on which the at least one further electrode (120) is located does not result in overlap between the geometrical projection of the site of the working electrode (118) and the site of the at least one further electrode (120).

6. The analyte sensor (110) according to any one of claims 1 to 5, wherein the conductive layer (122, 124) comprises an electrically conductive carbon material.

7. The analyte sensor (110) according to any one of claims 1 to 6, wherein the silver comprising layer (126) comprises Ag/AgCl.

8. The analyte sensor (110) according to any one of claims 1 to 7, wherein the protective layer (128) is a hydrophobic layer.

9. The analyte sensor (110) according to any one of claims 1 to 8, wherein the protective layer (128) is a membrane (134) comprising a plurality of holes (136), wherein the holes (136) are designed to provide access to the silver comprising layer (126) for the at least one body fluid comprising the at least one analyte.

10. The analyte sensor (110) according to any one of claims 1 to 9, wherein the silver comprising layer (126) comprises an exposed cutting edge (132) which provides access to the silver comprising layer (126) for the at least one body fluid comprising the at least one analyte.

11. The analyte sensor (110) according to any one of claims 1 to 10, wherein the analyte sensor (110) is a flat sensor.

12. The analyte sensor (110) according to any one of claims 1 to 11, wherein the analyte sensor (110) is a partially implantable sensor.

13. A method (140) for producing an analyte sensor (110) for determining at least one analyte, in particular the analyte sensor (110) according to any one of claims 1 to 12, the method comprising the steps of:
a) providing a raw substrate (142);
b) applying a conductive layer (122, 124) on the raw substrate (142);
c) applying a silver comprising layer (126) in a manner that it partially covers the conductive layer (124);
d) applying a protective layer (128) in a manner that it covers
o the silver comprising layer (126); and
o a portion of the conductive layer (124) such that the portion of the conductive layer (124) covered by the protective layer (128) is of 20 % to 80 %;
e) preparing a working electrode (118); and
f) cutting the raw substrate (142) to obtain the analyte sensor (110).

14. The method (140) according to claim 13, wherein the protective layer (128) is applied in a manner that it fully covers the silver comprising layer (126) apart from an accessible area (130) which provides access to the silver comprising layer (126) for at least one body fluid comprising the at least one analyte, wherein the accessible area (130) comprises a plurality of holes (136),
- wherein the protective layer (128) comprises the plurality of the holes (136) which are designed to provide access to the silver comprising layer (126) for at least one body fluid comprising the at least one analyte; and/or
- wherein, after cutting the raw substrate (142), the silver comprising layer (126) has an exposed cutting edge (132) which provides access to the silver comprising layer (126) for at least one body fluid comprising the at least one analyte.

## Patentansprüche

1. Analytsensor (110) zum Bestimmen mindestens eines Analyten, umfassend
- ein Substrat (112);
- eine Arbeitselektrode (118) und eine leitfähige Schicht (122, 124), die sich an verschiedenen Stellen auf dem Substrat (112) befinden;
- eine Silber umfassende Schicht (126), welche die leitfähige Schicht (124) teilweise bedeckt; und
- eine Schutzschicht (128), welche
o die Silber umfassende Schicht (126) vollständig bedeckt, mit Ausnahme mindestens eines Bereichs (130), der für mindestens eine Körperflüssigkeit, umfassend den mindestens einen Analyten, zugänglich ist; und
o einen Teil der leitfähigen Schicht (124) bedeckt;
**dadurch gekennzeichnet, dass**
der von der Schutzschicht (128) bedeckte Teil der leitfähigen Schicht (124) 20 % bis 80 % beträgt.

2. Analytsensor (110) nach Anspruch 1, wobei die leitfähige Schicht (124), die Silber umfassende Schicht (126) und die Schutzschicht (128) mindestens eine weitere Elektrode (120) bilden, ausgewählt aus einer Gruppe, bestehend aus einer Gegenelektrode, einer Referenzelektrode und einer kombinierten Gegen-/Referenzelektrode.

3. Analytsensor (110) nach Anspruch 2, wobei die mindestens eine weitere Elektrode (120) eine einzige kombinierte Gegen-/Referenzelektrode ist oder umfasst.

4. Analytsensor (110) nach einem der Ansprüche 2 oder 3, wobei sich die Arbeitselektrode (118) auf einer ersten Seite (114) des Substrats (112) befindet und wobei sich die mindestens eine weitere Elektrode (120) auf einer zweiten Seite (116) des Substrats (112) befindet, wobei die erste Seite (114) und die zweite Seite (116) des Substrats (112) einander gegenüberliegend positioniert sind.

5. Analytsensor (110) nach Anspruch 4, wobei sich die Arbeitselektrode (118) und die mindestens eine weitere Elektrode (120) derart auf dem Substrat (112) befinden, dass eine geometrische Projektion einer Stelle der Arbeitselektrode (118) auf die zweite Seite (116) des Substrats (112), auf der sich die mindestens eine weitere Elektrode (120) befindet, nicht zu einer Überlappung zwischen der geometrischen Projektion der Stelle der Arbeitselektrode (118) und der Stelle der mindestens einen weiteren Elektrode (120) führt.

6. Analytsensor (110) nach einem der Ansprüche 1 bis 5, wobei die leitfähige Schicht (122, 124) ein elektrisch leitfähiges Kohlenstoffmaterial umfasst.

7. Analytsensor (110) nach einem der Ansprüche 1 bis 6, wobei die Silber umfassende Schicht (126) Ag/AgCl umfasst.

8. Analytsensor (110) nach einem der Ansprüche 1 bis 7, wobei die Schutzschicht (128) eine hydrophobe Schicht ist.

9. Analytsensor (110) nach einem der Ansprüche 1 bis 8, wobei die Schutzschicht (128) eine Membran (134) ist, die eine Vielzahl von Löchern (136) umfasst, wobei die Löcher (136) so ausgelegt sind, dass sie einen Zugang zu der Silber umfassenden Schicht (126) für die mindestens eine Körperflüssigkeit bereitstellen, umfassend den mindestens einen Analyten.

10. Analytsensor (110) nach einem der Ansprüche 1 bis 9, wobei die Silber umfassende Schicht (126) eine freiliegende Schneidkante (132) umfasst, die einen Zugang zu der Silber umfassenden Schicht (126) für die mindestens eine Körperflüssigkeit bereitstellt, umfassend den mindestens einen Analyten.

11. Analytsensor (110) nach einem der Ansprüche 1 bis 10, wobei der Analytsensor (110) ein Flachsensor ist.

12. Analytsensor (110) nach einem der Ansprüche 1 bis 11, wobei der Analytsensor (110) ein teilweise implantierbarer Sensor ist.

13. Verfahren (140) zum Herstellen eines Analytsensors (110) zum Bestimmen mindestens eines Analyten, insbesondere des Analytsensors (110) nach einem der Ansprüche 1 bis 12, wobei das Verfahren die folgenden Schritte umfasst:
a) Bereitstellen eines Rohsubstrats (142);
b) Aufbringen einer leitfähigen Schicht (122, 124) auf das Rohsubstrat (142);
c) Aufbringen einer Silber umfassenden Schicht (126) derart, dass sie die leitfähige Schicht (124) teilweise bedeckt;
d) Aufbringen einer Schutzschicht (128) derart, dass sie
o die Silber umfassende Schicht (126) bedeckt; und
o einen Teil der leitfähigen Schicht (124) derart bedeckt, dass der von der Schutzschicht (128) bedeckte Teil der leitfähigen Schicht (124) 20 % bis 80 % beträgt;
e) Herstellen einer Arbeitselektrode (118); und
f) Schneiden des Rohsubstrats (142), um den Analytsensor (110) zu erhalten.

14. Verfahren (140) nach Anspruch 13, wobei die Schutzschicht (128) derart aufgebracht wird, dass sie die Silber umfassende Schicht (126) vollständig bedeckt, mit Ausnahme eines zugänglichen Bereichs (130), der einen Zugang zu der Silber umfassenden Schicht (126) für mindestens eine Körperflüssigkeit, umfassend den mindestens einen Analyten, bereitstellt, wobei der zugängliche Bereich (130) eine Vielzahl von Löchern (136) umfasst,
- wobei die Schutzschicht (128) die Vielzahl der Löcher (136) umfasst, die so ausgelegt sind, dass sie einen Zugang zu der Silber umfassenden Schicht (126) für mindestens eine Körperflüssigkeit bereitstellen, umfassend den mindestens einen Analyten; und/oder
- wobei nach dem Schneiden des Rohsubstrats (142) die Silber umfassende Schicht (126) eine freiliegende Schneidkante (132) aufweist, die einen Zugang zu der Silber umfassenden Schicht (126) für mindestens eine Körperflüssigkeit bereitstellt, umfassend den mindestens einen Analyten.

## Revendications

1. Capteur d'analyte (110) pour déterminer au moins un analyte, comprenant,
- un substrat (112) ;
- une électrode de travail (118) et une couche conductrice (122, 124) situées sur des sites différents sur le substrat (112) ;
- une couche comprenant de l'argent (126) recouvrant partiellement la couche conductrice (124) ; et
- une couche protectrice (128) recouvrant
∘ la couche comprenant de l'argent (126) entièrement sauf au moins une zone (130) accessible à au moins un fluide corporel comprenant l'au moins un analyte ; et
∘ une partie de la couche conductrice (124) ;
**caractérisé en**
**ce que** la partie de la couche conductrice (124) recouverte par la couche protectrice (128) représente de 20 % à 80 %.

2. Capteur d'analyte (110) selon la revendication 1, dans lequel la couche conductrice (124), la couche comprenant de l'argent (126) et la couche protectrice (128) forment au moins une électrode supplémentaire (120) choisie dans un groupe constitué par une contre-électrode, une électrode de référence et une combinaison de contre-électrode/électrode de référence.

3. Capteur d'analyte (110) selon la revendication 2, dans lequel l'au moins une électrode supplémentaire (120) est ou comprend une unique combinaison de contre-électrode/électrode de référence.

4. Capteur d'analyte (110) selon l'une quelconque des revendications 2 ou 3, dans lequel l'électrode de travail (118) est située sur un premier côté (114) du substrat (112) et dans lequel l'au moins une électrode supplémentaire (120) est située sur un second côté (116) du substrat (112), dans lequel le premier côté (114) et le second côté (116) du substrat (112) sont positionnés de manière opposée l'un à l'autre.

5. Capteur d'analyte (110) selon la revendication 4, dans lequel l'électrode de travail (118) et l'au moins une électrode supplémentaire (120) sont situées sur le substrat (112) de manière à ce qu'une projection géométrique d'un site de l'électrode de travail (118) sur le second côté (116) du substrat (112) sur lequel l'au moins une électrode supplémentaire (120) est située ne résulte pas en une superposition entre la projection géométrique du site de l'électrode de travail (118) et le site de l'au moins une électrode supplémentaire (120).

6. Capteur d'analyte (110) selon l'une quelconque des revendications 1 à 5, dans lequel la couche conductrice (122, 124) comprend un matériau carboné électriquement conducteur.

7. Capteur d'analyte (110) selon l'une quelconque des revendications 1 à 6, dans lequel la couche comprenant de l'argent (126) comprend de l'Ag/AgCl.

8. Capteur d'analyte (110) selon l'une quelconque des revendications 1 à 7, dans lequel la couche protectrice (128) est une couche hydrophobe.

9. Capteur d'analyte (110) selon l'une quelconque des revendications 1 à 8, dans lequel la couche protectrice (128) est une membrane (134) comprenant une pluralité d'orifices (136), dans lequel les orifices (136) sont conçus pour fournir un accès à la couche comprenant de l'argent (126) pour l'au moins un fluide corporel comprenant l'au moins un analyte.

10. Capteur d'analyte (110) selon l'une quelconque des revendications 1 à 9, dans lequel la couche comprenant de l'argent (126) comprend un bord de coupe (132) exposé qui fournit un accès à la couche comprenant de l'argent (126) pour l'au moins un fluide corporel comprenant l'au moins un analyte.

11. Capteur d'analyte (110) selon l'une quelconque des revendications 1 à 10, dans lequel le capteur d'analyte (110) est un capteur plat.

12. Capteur d'analyte (110) selon l'une quelconque des revendications 1 à 11, dans lequel le capteur d'analyte (110) est un capteur partiellement implantable.

13. Procédé (140) de production d'un capteur d'analyte (110) pour déterminer au moins un analyte, en particulier le capteur d'analyte (110) selon l'une quelconque des revendications 1 à 12, le procédé comprenant les étapes de :
a) fourniture d'un substrat brut (142) ;
b) application d'une couche conductrice (122, 124) sur le substrat brut (142) ;
c) application d'une couche comprenant de l'argent (126) de manière à ce qu'elle recouvre partiellement la couche conductrice (124) ;
d) application d'une couche protectrice (128) de manière à ce qu'elle recouvre
∘ la couche comprenant de l'argent (126) ; et
∘ une partie de la couche conductrice (124) de telle sorte que la partie de la couche conductrice (124) recouverte par la couche protectrice (128) représente de 20 % à 80 % ;
e) préparation d'une électrode de travail (118) ; et
f) découpe du substrat brut (142) pour obtenir le capteur d'analyte (110).

14. Procédé (140) selon la revendication 13, dans lequel la couche protectrice (128) est appliquée de manière à ce qu'elle recouvre entièrement la couche comprenant de l'argent (126) sauf une zone accessible (130) qui fournit un accès à la couche comprenant de l'argent (126) pour au moins un fluide corporel comprenant l'au moins un analyte, dans lequel la zone accessible (130) comprend une pluralité d'orifices (136),
- dans lequel la couche protectrice (128) comprend la pluralité d'orifices (136) qui sont conçus pour fournir un accès à la couche comprenant de l'argent (126) pour au moins un fluide corporel comprenant l'au moins un analyte ; et/ou
- dans lequel, après la découpe du substrat brut (142), la couche comprenant de l'argent (126) a un bord de coupe (132) exposé qui fournit un accès à la couche comprenant de l'argent (126) pour au moins un fluide corporel comprenant l'au moins un analyte.
